# EUROPEAN PATENT SPECIFICATION

(11) **EP 3 954 278 B1**
(45) Date of publication and mention of the grant of the patent: **25.11.2026**
(21) Application number: 20812876.9
(22) Date of filing: 27.05.2020
(51) Int. Cl.: A61B 5/00

(54) **APNEA MONITORING METHOD AND DEVICE**
VERFAHREN UND VORRICHTUNG ZUR ÜBERWACHUNG VON APNOE
PROCÉDÉ ET DISPOSITIF DE SURVEILLANCE DE L'APNÉE

(30) Priority: 31.05.2019 CN 201910471804
(43) Date of publication of application: 16.02.2022
(73) Proprietor: Huawei Technologies Co., Ltd., Shenzhen, Guangdong 518129 (CN)
(72) Inventor: HUANG, Xiaoping, Shenzhen, Guangdong 518129 (CN)
(74) Representative: Isarpatent
(86) International application number: PCT/CN2020/092597
(87) International publication number: WO 2020/238954

(56) References cited:
- WO-A1-2012/042453
- WO-A1-99/52438
- CN-A- 104 378 570
- CN-A- 105 662 417
- CN-A- 105 662 417
- CN-A- 106 913 335
- CN-A- 108 392 186
- CN-A- 108 419 168
- CN-A- 108 962 272
- CN-A- 109 300 475
- CN-A- 109 300 475
- CN-A- 109 805 895
- CN-A- 109 805 895
- CN-A- 110 301 890
- CN-B- 102 429 662
- CN-B- 105 962 894
- CN-U- 201 639 751
- US-A- 6 142 952
- US-A1- 2013 184 601
- US-A1- 2015 297 131
- US-A1- 2016 187 856
- US-A1- 2017 243 578
- US-B2- 6 882 971

## Description

Priority is claimed to Chinese Patent Application No. 201910471804.2, filed with the China National Intellectual Property Administration on May 31, 2019 and entitled "APNEA MONITORING METHOD AND APPARATUS".

### TECHNICAL FIELD

This disclosure generally relates to the field of medical monitoring, and the invention more specifically relates to an apnea monitoring method and terminal.

### BACKGROUND

Under the influence of factors such as increasing stress in modern society, overweight, and an irregular lifestyle, more than 20% of the population have a sleep apnea or hypopnea symptom. In daily life, because a user suffering from a sleep breathing disorder does not easily and autonomously perceive an occurrence time and severity that are of apnea, it may result in that the symptom cannot be treated and alleviated in a timely manner, consequently posing a great threat to health of the user.

Currently, a plurality of sleep breathing monitoring instruments may be used to monitor a sleep process, so as to help a user learn of whether an apnea disorder exists in the sleep process. A commonly used sleep breathing monitoring method is: using polysomnography (polysomnography, PSG) to diagnose obstructive sleep apnea; using a household sleep apnea screener to monitor an obstructive sleep apnea symptom of a user; or using an oximeter to monitor a change in blood oxygen of a user during sleep, to assist in monitoring an apnea condition of the user. However, all the currently used instruments need to be worn by a user, resulting in poor comfort, or monitoring needs to be performed in a hospital, and it takes a long time to obtain a report.

US 2015/0297131A1 discloses the use of a fast beam former and a slow beam former to process outputs of a microphone array so as to each form a directional beam such that sounds received from the directions inside the beam are enhanced. The fast beam former and the slow beam former focus the direction of their beams on the patient's head and adapt the direction of their beams based on movements of the patients head and body. Thus, the slow beam former and the fast beam former can track movements of the head and normal body movements of the patient with the beam.

WO 99/52438 discloses the evaluation of a breath sound sensor signal by performing an FFT transform and then a spectrum portion selection.

US 2013/0184601A1 relates to an apparatus and method for diagnosing obstructive sleep apnea. This document discloses a reference to a Fourier transform in connnection with a single person. The disclosure here is for the evaluation of a single signal from a single user.

US 2017/243578A1 relates to a voice processing method and device and US 6 882 971 B2 relates to a method and apparatus for improving listener differentiation of talkers during a conference call.

US 2016/0187856A1 relates to an alarm notification method and an apparatus, which implements a directional sound analysis and alarm notification.

### SUMMARY

The object of the present invention is to provide an apnea monitoring method and terminal, so as to resolve problems of poor comfort caused by a need to wear an instrument during sleep breathing monitoring and inability to implement simultaneous sleep breathing monitoring for a plurality of persons. This object is solved by the attached independent claims and further embodiments and improvements of the invention are listed in the attached dependent claims.

According to a first aspect of the disclosure, an apnea monitoring method is provided, including: collecting a first sound of at least one user by using a multi-microphone array of a first terminal, where the at least one user is located in coverage areas of different beams of the multi-microphone array; obtaining, based on the first sound, a power spectrum density corresponding to the first sound; and when the power spectrum density is less than a first threshold, determining that the at least one user encounters apnea.

It should be understood that the multi-microphone array may have directional beam forming through setting, and when the at least one user is separately located in the coverage areas of the directional beams of the multi-microphone array, microphones may collect a good audio signal of the first sound of the at least one user, to facilitate subsequent analysis of sleep apnea conditions of the at least one user by using the audio signal.

With reference to the first aspect, in some implementations of the first aspect, the collecting a first sound of at least one user by using a multi-microphone array of a first terminal includes: setting a sound pickup parameter of the multi-microphone array based on locations of the first terminal and the at least one user, so that the different beams of the multi-microphone array cover the at least one user; and separately collecting, by the multi-microphone array, the first sound of the at least one user in the coverage areas of the different beams.

According to the apnea monitoring method provided in this embodiment of this application, the first sound of the at least one user located in the coverage areas of the different directional beams of the multi-microphone array are collected by using the multi-microphone array of the first terminal, so that the first sound of the at least one user can be distinguished from each other based on the beam coverage areas in which the at least one user is located and based on a beam corresponding to a source of the first sound, and further, an apnea condition of the at least one user can be determined based on the first sound of the at least one user.

With reference to the first aspect, in some implementations of the first aspect, the obtaining, based on the first sound, a power spectrum density corresponding to the first sound includes: obtaining a time domain signal of the first sound based on the first sound; and performing Fourier transformation on the time domain signal to obtain the power spectrum density of the first sound.

It should be understood that a microphone of the first terminal may obtain the time domain signal of the first sound based on the collected first sound, and to facilitate intuitive analysis of an audio signal of the first sound, Fourier transformation may be performed to convert the time domain signal into the power spectrum density.

With reference to the first aspect, in some implementations of the first aspect, the method further includes: collecting, in advance, ambient noise of an environment in which the at least one user is located, and determining a frequency bandwidth of the ambient noise; and filtering out the ambient noise based on the frequency bandwidth of the ambient noise.

According to the apnea monitoring method provided in this embodiment of this application, the ambient noise of the environment in which the monitored user is located is filtered out, thereby obtaining a sleep sound of the at least one user in a better manner, and reducing interference caused by the ambient noise to a process of analyzing the sleep sound of the at least one user, where the sleep sound may be a breathing sound or a snoring sound.

With reference to the first aspect, in some implementations of the first aspect, the method further includes: determining, by using a voiceprint recognition algorithm, a first sound separately corresponding to each of the at least one user.

It should be understood that when sleep apnea monitoring is performed simultaneously on a plurality of users, first sounds of the different users can be more accurately distinguished from each other by using the voiceprint recognition algorithm, to facilitate subsequent analysis of sleep apnea conditions of the different users.

With reference to the first aspect, in some implementations of the first aspect, the method further includes: determining sleep duration of the at least one user; and determining an apnea-hypopnea index AHI of the at least one user based on the sleep duration and a quantity of times the at least one user encounters apnea in a sleep process.

According to the apnea monitoring method provided in this embodiment of this application, the AHI index of the at least one user is determined based on the sleep duration and the quantity of times the at least one user encounters apnea in the complete sleep process, thereby determining apnea severity of the at least one user more intuitively and more accurately.

According to a second but not claimed aspect, an apnea monitoring method is provided, including: collecting first sounds of a plurality of users by using a first terminal; determining, by using a voiceprint recognition algorithm, a first sound separately corresponding to each user; obtaining, based on the first sound, a power spectrum density corresponding to the first sound; and when the power spectrum density is less than a first threshold, determining that the user encounters apnea.

According to the apnea monitoring method provided in this non-claimed embodiment of this application, when sleep apnea monitoring is performed simultaneously on a plurality of users, first sounds of the different users can be more accurately distinguished from each other by using the voiceprint recognition algorithm, thereby enabling more accurate analysis of sleep apnea conditions of the different users.

With reference to the second aspect, in some implementations of the second aspect, the first terminal has a multi-microphone array. The collecting first sounds of a plurality of users by using a first terminal includes: collecting the first sounds of the plurality of users by using the multi-microphone array of the first terminal. The users are located in coverage areas of different beams of the multi-microphone array.

It should be understood that the multi-microphone array may have directional beam forming through setting, and when the users are separately located in coverage areas of directional beams of the multi-microphone array, microphones may collect good audio signals of the first sounds of the users, to facilitate subsequent analysis of sleep apnea conditions of the users by using the audio signals.

With reference to the second aspect, in some implementations of the second aspect, the collecting the first sounds of the plurality of users by using the multi-microphone array of the first terminal includes: setting a sound pickup parameter of the multi-microphone array based on locations of the first terminal and the users, so that the different beams of the multi-microphone array cover the different users; and separately collecting, by the multi-microphone array, the first sounds of the users in the coverage areas of the different beams.

According to the apnea monitoring method provided in this embodiment of this application, the first sounds of the users located in the coverage areas of the different directional beams of the multi-microphone array are collected by using the multi-microphone array of the first terminal, so that the first sounds of the different users can be distinguished from each other based on the beams in which the users are located and based on beams corresponding to sources of the first sounds, and further, apnea conditions of the users can be determined based on the sounds of the different users. According to the method in this embodiment, sleep apnea monitoring for one or more users can be implemented without a need for the one or more users to wear a monitoring instrument.

With reference to the second aspect, in some implementations of the second aspect, the obtaining, based on the first sound, a power spectrum density corresponding to the first sound includes: obtaining a time domain signal of the first sound based on the first sound; and performing Fourier transformation on the time domain signal to obtain the power spectrum density of the first sound.

It should be understood that a microphone may obtain the time domain signal of the first sound based on the collected first sound, and to facilitate intuitive analysis of an audio signal of the first sound, Fourier transformation may be performed to convert the time domain signal into the power spectrum density.

With reference to the second aspect, in some implementations of the second aspect, the method further includes: determining sleep duration of the user; and determining an AHI index of the user based on the sleep duration and a quantity of times the user encounters apnea in a sleep process.

According to the apnea monitoring method provided in this embodiment of this application, the ambient noise of the environment in which the monitored user is located is filtered out, thereby obtaining a sleep sound of the user in a better manner, and reducing interference caused by the ambient noise to a process of analyzing the sleep sound of the user, where the sleep sound may be a breathing sound or a snoring sound.

According to a third aspect of the disclosure, an apnea monitoring terminal is provided, including: a sound collection unit, configured to collect a first sound of at least one user; and a data processing unit, configured to obtain a power spectrum density of the first sound based on the first sound. The data processing unit is further configured to: when the power spectrum density is less than a first threshold, determine that the at least one user encounters apnea.

With reference to the third aspect, in some implementations of the third aspect, the obtaining a power spectrum density of the first sound based on the first sound includes: obtaining a time domain signal of the first sound based on the first sound; and performing Fourier transformation on the time domain signal to obtain the power spectrum density of the first sound.

With reference to the third aspect, in some implementations of the third aspect, the sound collection unit is further configured to: collect, in advance, ambient noise of an environment in which the at least one user is located; and the data processing unit is configured to: determine a frequency bandwidth of the ambient noise, and filter out the ambient noise based on the frequency bandwidth of the ambient noise.

With reference to the third aspect, in some implementations of the third aspect, the data processing unit is further configured to determine, by using a voiceprint recognition algorithm, a first sound separately corresponding to each of the at least one user.

With reference to the third aspect, in some implementations of the third aspect, the data processing unit is further configured to: determine sleep duration of the at least one user; and determine an AHI index of the at least one user based on the sleep duration and a quantity of times the at least one user encounters apnea in a sleep process.

According to a fourth aspect of the disclosure, an apnea monitoring apparatus is provided, including at least one microphone and a processor. The apparatus is used to perform the method according to the first aspect.

According to the apnea monitoring method and terminal provided in the embodiments of this application, sound information of the at least one user is distinguished from each other by using directional multi-beam forming of the multi-microphone array or voiceprint recognition, thereby implementing simultaneous monitoring of apnea conditions of a plurality of users. In addition, the users do not need to wear an instrument, thereby causing no impact on sleep.

In the following description, features which in the above summary of the invention have been marked as "not claimed" or "according to the invention" are also hereinafter, when they are described and explained with reference to the drawings, to be understood as "not claimed" or "not part of the invention" or "according to the invention". Even if sometimes in the description of the embodiments below, features marked above "according to the invention" or "the invention" are referred to in connection with the words "can" or "may" or other expressions which contain the notion of them being "optional", it should be understood that indeed such features are considered essential to the invention as claimed and not optional.

### BRIEF DESCRIPTION OF DRAWINGS

FIG. 1 shows a possible application scenario of an apnea monitoring method according to this application;
FIG. 2 is a schematic flowchart of an apnea monitoring method according to an embodiment of this application;
FIG. 3 is a schematic flowchart of another apnea monitoring method according to an embodiment of this application;
FIG. 4 is a schematic flowchart of still another apnea monitoring method according to an embodiment of this application;
FIG. 5 is a schematic flowchart of still another apnea monitoring method according to an embodiment of this application;
FIG. 6 is a schematic structural diagram of an apnea monitoring apparatus according to an embodiment of this application; and
FIG. 7 is a schematic structural diagram of another apnea monitoring apparatus according to an embodiment of this application.

### DESCRIPTION OF EMBODIMENTS

The following describes technical solutions of this application with reference to the accompanying drawings.

An obstructive sleep apnea syndrome (obstructive sleep apnea hypopnea syndrome, OSAHS) usually indicates that there are more than 30 times an airflow in a mouth or nose stops flowing for 10 seconds or more within a normal sleep period per night of an adult (for example, 7 hours). Currently, conventional methods for screening for and diagnosing obstructive sleep apnea include polysomnography PSG or performing monitoring by using an instrument such as a household sleep apnea screener or an oximeter. The following briefly describes user sleep monitoring methods currently used.
1. Using polysomnography PSG to diagnose obstructive sleep apnea.

PSG is a "gold standard" for diagnosing a sleep apnea syndrome. While monitoring obstructive sleep apnea, PSG can also identify many sleep-related diseases. A monitoring process of PSG is mainly as follows: During an examination, a doctor sticks some electrodes on an examinee, connects the electrodes to an instrument, and determines, by checking airflows in a mouth and nose of the user, a snoring sound of the user, and chest-abdominal exercises of the user, whether an apnea or hypopnea condition occurs. In addition, PSG can further obtain a maximum apnea period and an average apnea period, so as to distinguish between central apnea, obstructive apnea, and mixed apnea. When performing obstructive sleep apnea monitoring for a user, PSG requires use of a specific PSG instrument. In addition, monitoring is cumbersome, there is an excessively strict restriction on an environment of a place at which detection is performed, and the user has comparatively poor sleep experience. Therefore, it is difficult to implement convenient monitoring in daily life.

2. Using a household sleep apnea screener to monitor user sleep.

In view of complexity of PSG and inconvenience of using PSG, currently, there is still a portable household sleep apnea screener that retains a core monitoring capability of PSG but has a miniaturized structure. The household sleep apnea screener adopts a principle of monitoring a user for apnea that is similar to a principle of PSG. The household sleep apnea screener also needs to be worn by a user, and therefore, also causes comparatively poor user experience. Besides, an effect of simultaneously monitoring a plurality of persons by using a same instrument may also not be achieved.

3. Using an oximeter to assist in screening for sleep apnea.

When a user encounters obstructive apnea, it causes a drop in blood oxygen. Therefore, an objective of assisting in screening for a sleep apnea syndrome may be achieved by monitoring blood oxygen of the user during sleep. However, in an actual application process, because a drop in blood oxygen may be caused by a plurality of reasons, for example, a heart failure, the oximeter-assisted monitoring method cannot accurately identify a cause of the drop in blood oxygen, and therefore, causes a comparatively large error in a monitoring result.

In view of disadvantages of the current methods for monitoring an obstructive sleep apnea syndrome of a user, an embodiment of this application provides a method that does not require a user to wear an instrument and can implement simultaneous sleep monitoring for a plurality of persons by using one apparatus. The following describes, with reference to the accompanying drawings, the sleep monitoring method provided in this application.

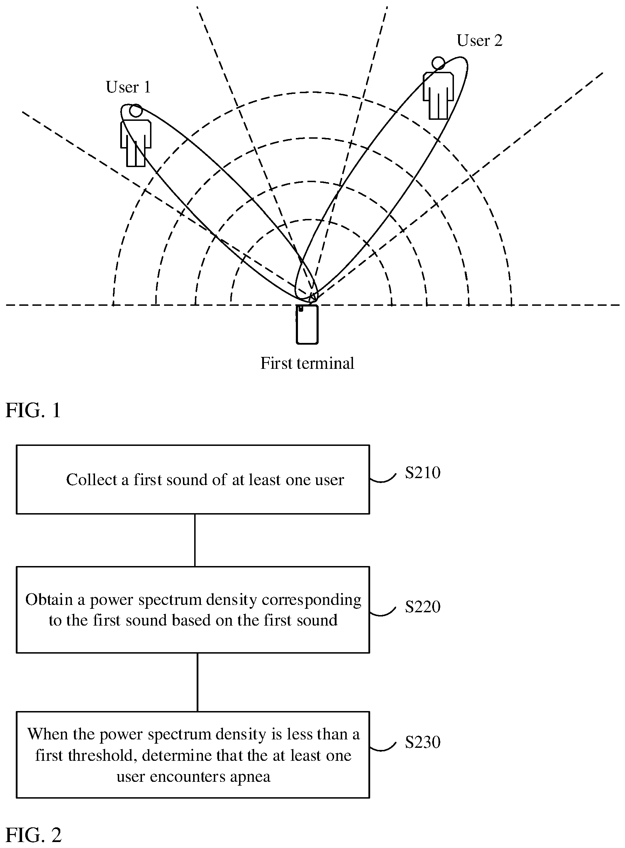

FIG. 1 shows a possible application scenario of an apnea monitoring method according to this application.

First, it should be understood that a microphone may have an omnidirectional sound pickup response, that is, the microphone may respond to sounds from all directions, and a multi-microphone array formed through combination of a plurality of microphones may form a directional response or a beam field pattern. Through design, the microphone array may be more sensitive to sounds from one or more specific directions, and can collect a better sound signal in the specific direction.

According to the sleep monitoring method provided in this embodiment of this application, audio signals of sleep sounds of users located in different positions are collected by using a beam forming technology of the multi-microphone array, thereby implementing simultaneous monitoring of sleep processes of a plurality of users without a need for the users to wear an instrument, and further determining whether the plurality of users encounter obstructive sleep apnea or determining severity of obstructive sleep apnea. The sleep sound may be, for example, a breathing sound or a snoring sound of a user during sleep. In addition, the multi-microphone array may be a multi-microphone array in an existing device, that is, an existing terminal device equipped with a multi-microphone array may be used to collect sleep sounds of a plurality of users. The terminal device may be, for example, a mobile phone or a recording pen.

FIG. 2 is a schematic flowchart of an apnea monitoring method according to an embodiment of this application. The method includes the following steps.

S210: Collect a first sound of at least one user.

A first terminal equipped with a microphone may be used to collect the first sound of the monitored user. The first terminal may be, for example, a mobile phone equipped with a single microphone or a multi-microphone array or another recording device equipped with a single microphone or a multi-microphone array. The first sound is a sleep sound of the at least one user during sleep, and may be, for example, a breathing sound or a snoring sound of the at least one user during sleep.

It should be understood that the apnea monitoring method provided in this embodiment of this application may be used for simultaneous monitoring of sleep breathing conditions of a plurality of users. Therefore, when sleep sounds of a plurality of users are collected, to implement subsequent separate analysis of a sleep apnea condition of each user, a sleep sound corresponding to each user needs to be identified. In this embodiment of this application, sleep sounds of different users are distinguished from each other mainly in two manners. In a first manner, a first terminal equipped with a multi-microphone array is used to collect first sounds of a plurality of users, where the first sounds of the users located in areas of different beams are collected based on directional beam forming of the multi-microphone array; and then a correspondence between a user and a first sound is determined based on a source of the first sound and a location of the user. In a second manner, after first sounds of a plurality of users are collected by using a first terminal, a first sound of each user is determined by using a voiceprint recognition algorithm.

For example, the first device equipped with a multi-microphone array is used to record the first sound of the at least one user. It should be understood that by setting a sound pickup parameter of the microphone array, for example, a sound pickup direction or a sound pickup included-angle width, in combination with locations of the first device and the at least one user, the at least one user can be enabled to be in a coverage area of a beam of the multi-microphone array. In addition, phase adjustment is performed on signals of microphone array units, so that the phase-adjusted signals of the units are superimposed to obtain a main lobe signal in a user-specified direction, thereby enabling the first device to accurately collect first sounds of users located in different positions.

It should be understood that when sleep breathing processes of a plurality of users are simultaneously monitored, because different users are separately located in coverage areas of different beams of a multi-microphone array, a correspondence between a first sound and a user may be determined based on a beam coverage area corresponding to a source of the collected first sound. That is, when a plurality of first sounds of a plurality of users are collected, a first sound corresponding to each user may be determined based on beam coverage areas in which the plurality of users are located and based on beams corresponding to sources of the plurality of first sounds, to facilitate subsequent separate analysis of apnea conditions of the different users based on the first sounds of the different users.

Optionally, the first device equipped with a multi-microphone array may be a mobile phone with a multi-microphone array, a recording pen with a multi-microphone array, or another terminal device with a multi-microphone array. This is not limited in this application.

For another example, after the first sounds of the users are collected by using the first device, the first sound of each user is determined by using the voiceprint recognition algorithm. It should be understood that a voiceprint (voiceprint) is a sound wave spectrum that carries verbal information and that is displayed by an electro acoustic instrument. Because vocal organs vary greatly in size and form when people make a sound, voiceprint graphs of any two persons are different. Usually, based on voiceprint information, different persons may be distinguished from each other or it may be determined whether collected sounds belong to a same person. In this embodiment, a process of determining, in a voiceprint recognition manner, a first sound corresponding to a user may be, for example, as follows: during formal sleep breathing monitoring performed on monitored users, collecting a first sound of each user, and obtaining a short-time phonetic spectrum of the sound of each user; extracting a voiceprint characteristic based on the short-time phonetic spectrum; and determining, by using a voiceprint recognition model and the voiceprint characteristics of the users, a user corresponding to the first sound. The voiceprint recognition model may be an existing model, for example, may be a Gaussian mixture model (Gaussian mixture model, GMM), a support vector machine (support vector machine, SVM) model, a channel model, or an identity vector (identity vector, i-vector) model.

A voiceprint recognition process is further described by using the GMM model as an example.

It should be understood that a difference between voices of different persons is mainly reflected by a difference between short-time phonetic spectra, and the difference between short-time phonetic spectra may be measured by probability density functions of the short-time phonetic spectra. In the GMM model, a probability density of spatial distribution may be obtained through fitting of weighted sums of a plurality of Gaussian probability density functions. A Gaussian probability density function obtained through fitting may smoothly approximate a probability density function in any shape, and is an easy-to-process parameter model. In specific representation, the parameter model may be a model that uses, as a specific person making a sound, a super vector formed by arranging mean vectors of Gaussian components of the GMM model together.

For example, the voiceprint recognition model in this embodiment may be a GMM model that is based on a mel-frequency cepstral coefficient (Mel frequency cepstrum coefficient, MFCC). A process of performing voiceprint recognition by using the GMM model may include: (1) GMM model training and (2) a voiceprint recognition process. During GMM training, a plurality of voice signals may be collected; characteristic parameters of the voice signals may be extracted after the plurality of voice signals are preprocessed, where the voice characteristic parameter may be, for example, an MFCC; and the GMM model is trained by using the characteristic parameters of the voice signals as samples. In the voiceprint recognition process, a characteristic parameter of the collected first sound is obtained based on the first sound, the characteristic parameter of the first sound is compared with the built GMM model, and a user corresponding to the first sound is determined based on a recognition accuracy rate of the GMM.

It should be understood that the voiceprint recognition process used in this embodiment of this application may alternatively be another existing process, and is not limited to the voiceprint recognition manner mentioned above.

It should be understood that during actual recording of a sleep sound of a user, ambient noise inevitably exists. Therefore, to obtain an accurate and good sleep sound signal for a subsequent analysis process, the ambient noise needs to be eliminated.

For example, in this embodiment of this application, a process of eliminating or reducing the ambient noise may be as follows: Before a sleep sound signal of a user is formally collected, ambient noise of an environment in which the user is located is collected, to obtain a phase, a frequency, an amplitude, and the like that correspond to a sound wave of the ambient noise. During subsequent sound recording performed for user insomnia monitoring, the plurality of microphones generate a sound wave whose phase is opposite to the phase of the sound wave of the pre-recorded ambient noise and whose frequency and amplitude are the same as the frequency and the amplitude that are of the sound wave of the pre-recorded ambient noise, to make the sound wave interfere with the ambient noise to implement phase cancellation, thereby eliminating the ambient noise. Alternatively, a filter frequency bandwidth of a filter is set based on a frequency bandwidth of the pre-recorded ambient noise, so that the filter can filter out the ambient noise during recording. A manner of filtering out the ambient noise may alternatively be another existing manner, and this is not limited in this application.

It should be understood that the first sound of the at least one user during sleep is recorded by a microphone, and then a sleep apnea condition of the at least one user is analyzed, so that the at least one user can be monitored for sleep apnea without a need to wear a monitoring instrument, thereby improving the at least one user's comfort in a monitoring process.

Step S220: Obtain, based on the first sound, a power spectrum density corresponding to the first sound.

Usually, a sound signal obtained by a microphone is a time domain signal. For more intuitive analysis of a sound signal of a user, the time domain signal is converted into a frequency domain signal. Specifically, Fourier transformation is performed on a time domain signal of the obtained sleep sound of the at least one user to obtain a power spectrum corresponding to the time domain signal, that is, a power spectrum density. A formula used in a Fourier transformation process is: $F \left(ω\right) = F \left[f\left(t\right)\right] = {\int }_{- ∞}^{∞} f \left(t\right) {e}^{- iωt} dt$, where ω represents a frequency, t represents a time, *e*^{-*iωt*} is a complex-variable function, f(t) may be a time domain signal of the first sound, and F(ω) may be a frequency domain signal obtained after Fourier transformation is performed.

S230: When the power spectrum density is less than a first threshold, determine that the at least one user encounters apnea.

When the power spectrum density of the sleep sound of the at least one user is less than the first threshold, it is determined that the at least one user encounters apnea.

It should be understood that the first threshold may be a value that is set based on a normal breathing state of the at least one user. For example, a process of obtaining the first threshold may be as follows: performing time-frequency conversion on a time-frequency signal of the sleep sound of the at least one user who is in the normal breathing state (for example, a breathing state in which the at least one user has just fallen asleep and does not encounter apnea), to obtain a frequency spectrum of a breathing sound in the state; calculating an average value of a plurality of (for example, five to ten) consecutive breathing spectrum peak values; and then taking 30% to 50% of the average value as the first threshold, used for subsequently determining whether the at least one user encounters apnea and determining apnea severity. In addition, the first threshold may be optimized and adjusted based on clinical test data and with reference to PSG standard precision, to achieve optimal sensitivity and specificity.

Optionally, when duration during which the power spectrum density of the sleep sound of the at least one user is less than the first threshold reaches a second threshold, it is determined that the at least one user encounters apnea once. The second threshold may be, for example, 10s.

Optionally, an apnea-hypopnea index (apnea-hypopnea index, AHI) of the at least one user is determined based on a quantity of times the power spectrum density of the first sound of the at least one user is less than the first threshold. Specifically, the AHI index of the at least one user may be determined based on sleep duration of the at least one user and the quantity of times the power spectrum density of the first sound is less than the first threshold.

Optionally, when sleep of the at least one user is monitored, a sleep period of the at least one user may be recorded. For example, the sleep period may be sleep duration of the at least one user throughout a night.

For example, the AHI index of the at least one user is determined based on a power spectrum density of the breathing sound of the at least one user, and/or a power spectrum density of the snoring sound of the at least one user, and the sleep period of the at least one user. For example, when the power spectrum density of the breathing sound of the at least one user or the power spectrum density of the snoring sound of the at least one user is less than the first threshold, and duration during which the power spectrum density of the breathing sound of the at least one user or the power spectrum density of the snoring sound of the at least one user is less than the first threshold reaches the second threshold, it is recorded that apnea occurs once. In an entire sleep process of the at least one user, a total quantity of times (denoted as k) the at least one user encounters apnea is recorded, and the sleep apnea-hypopnea index is calculated based on entire sleep duration (denoted as t) of the at least one user, where AHI=k/t. Alternatively, the following value is recorded: a quantity of times per unit time the power spectrum density of the sleep sound of the at least one user is less than the first threshold for duration that reaches the second threshold.

Severity of apnea that a user encounters is determined based on an AHI index of the user. For example, when the AHI is within a range less than or equal to 5, it is determined that the user has normal sleep, that is, it is not considered that apnea exists; when a value of the AHI is within a range of 5 to 15, it is considered that the user has a mild sleep apnea condition; when a value of the AHI is within a range of 15 to 30, it is considered that the user has a moderate sleep apnea condition; or when the AHI is within a range greater than or equal to 30, it is considered that the user has a severe sleep apnea condition.

According to the apnea monitoring method provided in this application, sound information of different users is distinguished from each other by using directional multi-beam forming of the multi-microphone array or voiceprint recognition, thereby implementing simultaneous monitoring of apnea conditions of a plurality of users. In addition, the users do not need to wear an instrument, thereby causing no impact on sleep.

The following specifically describes an apnea monitoring method in this application with reference to the accompanying drawings.

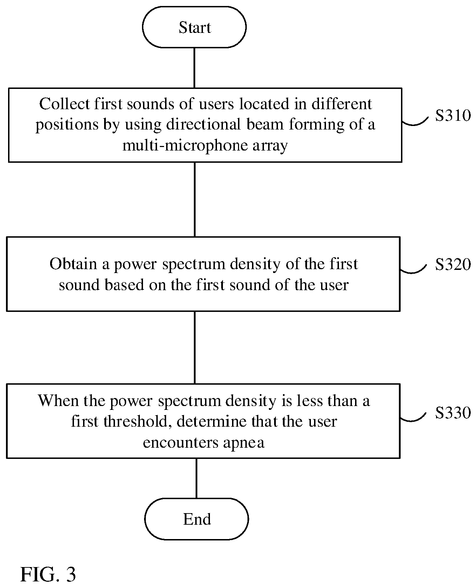

FIG. 3 is a schematic flowchart of an apnea monitoring method according to an embodiment of this application. The method includes the following steps.

S310: Collect, by using directional beam forming of a multi-microphone array, first sounds of users located in different positions.

A first terminal equipped with a microphone may be used to collect the first sounds of the monitored users. The first terminal may be, for example, a mobile phone equipped with a single microphone or a multi-microphone array or another recording device equipped with a single microphone or a multi-microphone array. The first sounds are sleep sounds of the users during sleep, and may be, for example, breathing sounds or snoring sounds of the users during sleep.

The plurality of monitored users may be separately located in coverage areas of directional beams of the multi-microphone array.

Optionally, before sleep breathing of the users is monitored, a sleep apnea monitoring function of the first device is enabled manually or in another manner.

Optionally, after collecting the first sounds of the plurality of users, the first terminal may further recognize the first sounds of the different users by using a voiceprint recognition algorithm, so as to distinguish between the first sounds of the different users more accurately.

For example, a process of recording the first sound of the at least one user by using the first device equipped with a multi-microphone array may include: setting a sound pickup parameter of the microphone array, for example, a sound pickup direction or a sound pickup included-angle width, based on locations of the first device and the users, so that the users are in the coverage areas of the beams of the multi-microphone array; and in addition, performing phase adjustment on signals of microphone array units, so that the phase-adjusted signals of the units are superimposed to obtain a main lobe signal in a user-specified direction, thereby enabling the first device to accurately collect the first sounds of the users located in different positions.

It should be understood that when sleep breathing processes of a plurality of users are simultaneously monitored, because different users are separately located in coverage areas of different beams of a multi-microphone array, a correspondence between a first sound and a user may be determined based on a beam coverage area corresponding to a source of the collected first sound. That is, when a plurality of first sounds of a plurality of users are collected, a first sound corresponding to each user may be determined based on beam coverage areas in which the plurality of users are located and based on beams corresponding to sources of the plurality of first sounds, to facilitate subsequent separate analysis of apnea conditions of the different users based on the first sounds of the different users.

It should be understood that during actual recording of a sleep sound of a user, ambient noise inevitably exists. Therefore, to obtain an accurate and good sleep sound signal for a subsequent analysis process, the ambient noise needs to be eliminated or reduced.

For example, in this embodiment of this application, a process of eliminating or reducing the ambient noise may be as follows: Before a sleep sound signal of a user is formally collected, ambient noise of an environment in which the user is located is collected, to obtain a phase, a frequency, an amplitude, and the like that correspond to a sound wave of the ambient noise. During subsequent sound recording performed for user insomnia monitoring, the plurality of microphones generate a sound wave whose phase is opposite to the phase of the sound wave of the pre-recorded ambient noise and whose frequency and amplitude are the same as the frequency and the amplitude that are of the sound wave of the pre-recorded ambient noise, to make the sound wave interfere with the ambient noise to implement phase cancellation, thereby eliminating the ambient noise. Alternatively, a filter frequency bandwidth of a filter is set based on a frequency bandwidth of the pre-recorded ambient noise, so that the filter can filter out the ambient noise during recording. A manner of filtering out the ambient noise may alternatively be another existing manner, and this is not limited in this application.

It should be understood that the first sounds of the users during sleep are recorded by microphones, and then sleep apnea conditions of the users are analyzed, so that the users can be monitored for sleep apnea without a need to wear a monitoring instrument, thereby improving the users' comfort in a monitoring process.

S320: Obtain a power spectrum density of the first sound based on the first sound of the user.

The multi-microphone array may obtain a time domain signal of the first sound based on the collected first sound.

Optionally, a signal in time domain may be converted into a power spectrum density in frequency domain through Fourier transformation. For a specific conversion process, refer to step S220. To avoid repetition, details are not described herein again.

S330: When the power spectrum density is less than a first threshold, determine that the user encounters apnea.

When the power spectrum density of the sleep sound of the user is less than the first threshold, it is determined that the user encounters apnea.

It should be understood that the first threshold may be a value that is set based on a normal breathing state of the user. For example, a process of obtaining the first threshold may be as follows: performing time-frequency conversion on a time-frequency signal of the sleep sound of the user who is in the normal breathing state (for example, a breathing state in which the user has just fallen asleep and does not encounter apnea), to obtain a frequency spectrum of a breathing sound in the state; calculating an average value of a plurality of (for example, five to ten) consecutive breathing spectrum peak values; and then taking 30% to 50% of the average value as the first threshold, used for subsequently determining whether the user encounters apnea and determining apnea severity. In addition, the first threshold may be optimized and adjusted based on clinical test data and with reference to PSG standard precision, to achieve optimal sensitivity and specificity.

Optionally, when duration during which the power spectrum density of the sleep sound of the user is less than the first threshold reaches a second threshold, it is determined that the user encounters apnea once. The second threshold may be, for example, 10s or 15s.

Optionally, an apnea-hypopnea index AHI of the user is determined based on a quantity of times the power spectrum density of the first sound of the user is less than the first threshold.

Optionally, an apnea-hypopnea index AHI of the user is determined based on the power spectrum density of the first sound of the user. Specifically, the AHI index of the user may be determined based on sleep duration of the user and the quantity of times the power spectrum density of the first sound is less than the first threshold.

Optionally, when sleep of the user is monitored, a sleep period of the user may be recorded. For example, the sleep period may be sleep duration of the user throughout a night.

For example, when the power spectrum density of the breathing sound of the user or the power spectrum density of the snoring sound of the user is less than the first threshold, and duration during which the power spectrum density of the breathing sound of the user or the power spectrum density of the snoring sound of the user is less than the first threshold reaches the second threshold, it is recorded that apnea occurs once. In an entire sleep process of the user, a total quantity of times (denoted as k) the user encounters apnea is recorded, and the sleep apnea-hypopnea index is calculated based on entire sleep duration (denoted as t) of the user, where AHI=k/t. Alternatively, the following value is recorded: a quantity of times per unit time the power spectrum density of the sleep sound of the user is less than the first threshold for duration that reaches the second threshold.

It should be understood that after the AHI index of the user is obtained, apnea severity of the user may be determined based on the AHI index. For example, when the AHI is within a range less than or equal to 5, it is determined that the user has normal sleep, that is, it is not considered that apnea exists; when a value of the AHI is within a range of 5 to 15, it is considered that the user has a mild sleep apnea condition; when a value of the AHI is within a range of 15 to 30, it is considered that the user has a moderate sleep apnea condition; or when the AHI is within a range greater than or equal to 30, it is considered that the user has a severe sleep apnea condition.

According to the apnea method provided in this embodiment of this application, the sleep sounds of the users are collected by using the multi-microphone array, thereby implementing non-contact sleep apnea monitoring and risk assessment for the plurality of users.

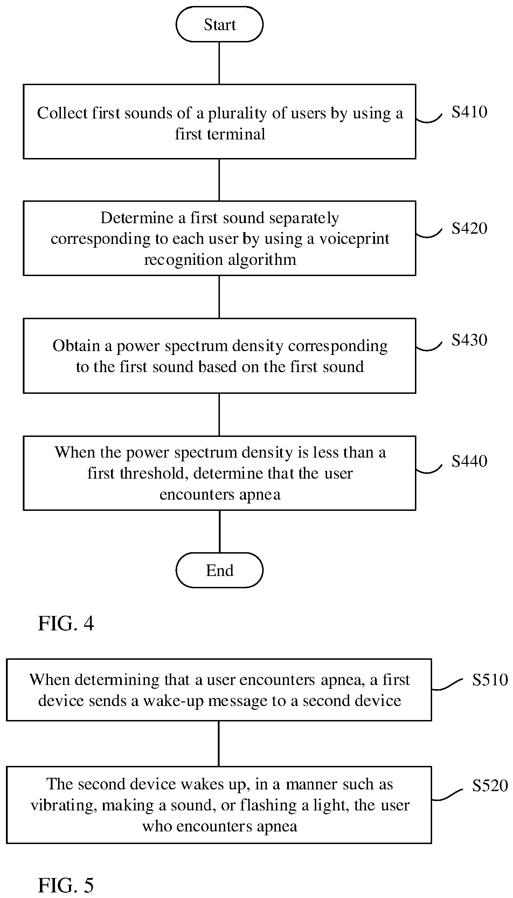

FIG. 4 is a schematic flowchart of another apnea monitoring method according to an embodiment of this application. The method includes the following steps.

S410: Collect first sounds of a plurality of users by using a first terminal.

Optionally, the first terminal may be a mobile phone, a recording pen, or the like, or may be another terminal device equipped with one or more microphones.

Optionally, the first sounds of the users are recorded by using a microphone in the first device. The first sounds are breathing sounds, snoring sounds, or the like of the users during sleep.

Optionally, the microphone in the first terminal may obtain a time domain signal of a collected first sound based on the first sound.

It should be understood that during actual recording of a sleep sound of a user, ambient noise inevitably exists. Therefore, to obtain an accurate and good sleep sound signal for a subsequent analysis process, the ambient noise needs to be eliminated or reduced.

For example, in this embodiment of this application, a process of eliminating or reducing the ambient noise may be as follows: Before a sleep sound signal of a user is formally collected, ambient noise of an environment in which the user is located is collected, to obtain a phase, a frequency, an amplitude, and the like that correspond to a sound wave of the ambient noise. During subsequent sound recording performed for user insomnia monitoring, the plurality of microphones generate a sound wave whose phase is opposite to the phase of the sound wave of the pre-recorded ambient noise and whose frequency and amplitude are the same as the frequency and the amplitude that are of the sound wave of the pre-recorded ambient noise, to make the sound wave interfere with the ambient noise to implement phase cancellation, thereby eliminating the ambient noise. Alternatively, a filter frequency bandwidth of a filter is set based on a frequency bandwidth of the pre-recorded ambient noise, so that the filter can filter out the ambient noise during recording. A manner of filtering out the ambient noise may alternatively be another existing manner, and this is not limited in this application.

It should be understood that the first sounds of the users during sleep are recorded by microphones, and then sleep apnea conditions of the users are analyzed, so that the users can be monitored for sleep apnea without a need to wear a monitoring instrument, thereby improving the users' comfort in a monitoring process.

S420: Determine, by using a voiceprint recognition algorithm, a first sound corresponding to each user.

Optionally, before the first sounds of the users during sleep are collected, that is, in a preprocessing phase, the breathing sounds or the snoring sounds of the users at a time sleep apnea does not occur are collected in advance. For example, segments of the breathing sounds and/or the snoring sounds of the users may be collected in early sleep phases of the users in which muscles of the users are not yet in a fully relaxed state and sleep apnea does not occur.

Optionally, sound characteristics of the first sounds of the users are extracted, and voiceprint models are built for the different users.

Optionally, the first sound of each user is determined based on the first sounds obtained in a formal monitoring process and the voiceprint models of the different users.

For example, after the first sounds of the users are collected by using the first device, the first sound of each user is determined by using the voiceprint recognition algorithm. It should be understood that a voiceprint (voiceprint) is a sound wave spectrum that carries verbal information and that is displayed by an electroacoustic instrument. Because vocal organs vary greatly in size and form when people make a sound, voiceprint graphs of any two persons are different. Usually, based on voiceprint information, different persons may be distinguished from each other or it may be determined whether collected sounds belong to a same person. In this embodiment, a process of determining, in a voiceprint recognition manner, a first sound corresponding to a user may be, for example, as follows: before formal sleep breathing monitoring is performed on monitored users, collecting a first sound of each user in advance, for example, a breathing sound or a snoring sound, and extracting a characteristic of the first sound of each user, for example, obtaining a short-time phonetic spectrum of the sound of each user; building a voiceprint recognition model based on the characteristic of the first sound of each user; and collecting the first sound of the user during sleep, and determining the first sound of each user based on the built voiceprint recognition model.

For example, a process of building a user voiceprint recognition model may be, for example, as follows: obtaining, based on the characteristics of the first sounds of the users, for example, the short-time phonetic spectra, probability density functions corresponding to the characteristics of the first sounds of the users; and building a Gaussian mixture model (Gaussian mixture model, GMM) to obtain a probability density of spatial distribution through fitting of weighted sums of a plurality of Gaussian probability density functions, so that the Gaussian mixture model can smoothly approximate a probability density function in any shape, to obtain an easy-to-process parameter model. Specifically, the parameter model obtained based on the probability density functions of the short-time phonetic spectra is a model that uses, as a specific user, a super vector formed by arranging mean vectors of Gaussian components of the Gaussian mixture model together. The first sound corresponding to each user may be determined based on a specific parameter model corresponding to each user. For a process of building a parameter model for each user based on voiceprint recognition, refer to an existing procedure, and details are not described herein again.

S430: Obtain, based on the first sound, a power spectrum density corresponding to the first sound.

Optionally, Fourier transformation is performed on a time domain signal obtained by a microphone, to obtain the power spectrum density of the first sound of each user.

S440: When the power spectrum density is less than a first threshold, determine that the user encounters apnea.

When the power spectrum density of the sleep sound of the user is less than the first threshold, it is determined that the user encounters apnea.

It should be understood that the first threshold may be a value that is set based on a normal breathing state of the user. For example, a process of obtaining the first threshold may be as follows: performing time-frequency conversion on a time-frequency signal of the sleep sound of the user who is in the normal breathing state (for example, a breathing state in which the user has just fallen asleep and does not encounter apnea), to obtain a frequency spectrum of a breathing sound in the state; calculating an average value of a plurality of (for example, five to ten) consecutive breathing spectrum peak values; and then taking 30% to 50% of the average value as the first threshold, used for subsequently determining whether the user encounters apnea and determining apnea severity. In addition, the first threshold may be optimized and adjusted based on clinical test data and with reference to PSG standard precision, to achieve optimal sensitivity and specificity.

Optionally, when duration during which the power spectrum density of the sleep sound of the user is less than the first threshold reaches a second threshold, it is determined that the user encounters apnea. The second threshold may be, for example, 10s or 15s.

Optionally, an apnea-hypopnea index AHI of the user is determined based on a quantity of times the power spectrum density of the first sound of the user is less than the first threshold. Specifically, the AHI index of the user is determined based on sleep duration of the user and a quantity of times the user encounters apnea. The AHI index may intuitively reflect sleep apnea severity of the user.

For example, when the power spectrum density of the breathing sound of the user or the power spectrum density of the snoring sound of the user is less than the first threshold, and duration during which the power spectrum density of the breathing sound of the user or the power spectrum density of the snoring sound of the user is less than the first threshold reaches the second threshold, it is recorded that apnea occurs once. In an entire sleep process of the user, a total quantity of times (denoted as k) the user encounters apnea is recorded, and the sleep apnea-hypopnea index is calculated based on entire sleep duration (denoted as t) of the user, where AHI=k/t. Alternatively, the following value is recorded: a quantity of times per unit time the power spectrum density of the sleep sound of the user is less than the first threshold for duration that reaches the second threshold.

For a manner of determining a sleep apnea condition of the user based on a value of the AHI index, refer to step S330. To avoid repetition, details are not described herein again.

According to the apnea method provided in this embodiment of this application, the first sounds of the different users are collected by using a microphone, and the sleep sounds corresponding to the different users are distinguished from each other by using the voiceprint recognition algorithm, thereby implementing non-contact sleep apnea monitoring and risk assessment for the plurality of users.

FIG. 5 is a schematic flowchart of an apnea monitoring method according to an embodiment of this application.

The apnea monitoring method in this embodiment of this application may further include the following steps.

S510: When determining that a user encounters apnea, a first device sends a wake-up message to a second device.

When it is determined that the user encounters apnea or remains in apnea for a specific period of time, the user is woken up.

Optionally, the second device may be a smart device around the user, for example, a smart speaker, a smart light, a smart wearable device, or a smartphone, or may be a smart device of an emergency contact of the user, for example, a smartphone.

Optionally, when it is detected that duration during which the user remains in apnea reaches a third threshold, the first device sends the wake-up message to the second device. The third threshold may be, for example, 20s.

S520: The second device wakes up, in a manner such as vibrating, making a sound, or flashing a light, the user who encounters apnea.

When receiving the wake-up message from the first device, the second device may wake up the user in different manners, for example, wake up, in a manner such as vibrating, making a sound, or flashing a light, the user who encounters apnea.

For example, when it is detected, by using the first device, that the duration during which the user remains in apnea reaches the third threshold, the first device may send the wake-up message to a linked device around the user, for example, a smart speaker, a smart light, a smart wearable device, or a smartphone, so that the smart device around the user wakes up the user timely in a corresponding manner such as vibrating, making a sound, or flashing a light. The third threshold may be, for example, 20s or another value that is set with reference to a doctor's suggestion. This is not limited in this application.

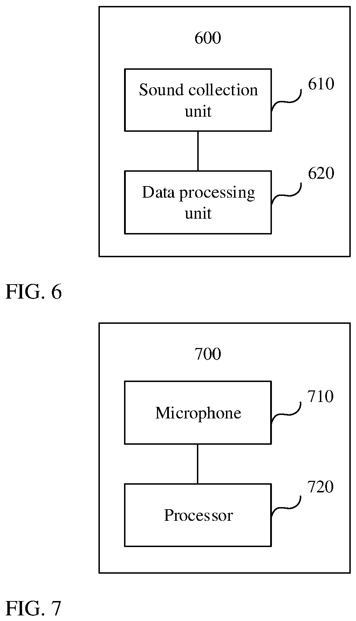

FIG. 6 shows a sleep apnea monitoring apparatus according to an embodiment of this application. The apparatus 600 includes a sound collection unit 610 and a data processing unit 620.

The sound collection unit 610 is configured to collect a first sound of at least one user.

Optionally, the data processing unit 620 is configured to obtain a power spectrum density of the first sound based on the first sound.

Optionally, the data processing unit 620 may be further configured to determine an AHI index of the at least one user based on the power spectrum density.

FIG. 7 shows another sleep apnea monitoring apparatus according to an embodiment of this application. The apparatus 700 includes at least one microphone 710 and a processor 720.

Optionally, the microphone 710 is configured to collect a first sound of at least one user.

Optionally, the processor 720 is configured to obtain a power spectrum density of the first sound based on the first sound.

Optionally, the processor 720 may be further configured to determine an AHI index of the at least one user based on the power spectrum density.

A person of ordinary skill in the art may be aware that, in combination with the examples described in the embodiments disclosed in this specification, units and algorithm steps may be implemented by electronic hardware or a combination of computer software and electronic hardware. Whether the functions are performed by hardware or software depends on particular applications and design constraints of the technical solutions. A person skilled in the art may use different methods to implement the described functions for each particular application, but it should not be considered that the implementation goes beyond the scope of this application.

It may be clearly understood by a person skilled in the art that, for the purpose of convenient and brief description, for a detailed working process of the foregoing system, device, and unit, refer to a corresponding process in the foregoing method embodiments, and details are not described herein again.

In the several embodiments provided in this application, it should be understood that the disclosed system, apparatus, and method may be implemented in other manners. For example, the described apparatus embodiment is merely an example. For example, the unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electrical, mechanical, or other forms.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions of the embodiments.

In addition, functional units in the embodiments of this application may be integrated into one processing unit, or each of the units may exist alone physically, or two or more units are integrated into one unit.

When the functions are implemented in the form of a software functional unit and sold or used as an independent product, the functions may be stored in a computer-readable storage medium. Based on such an understanding, the technical solutions of this application essentially, or the part contributing to the prior art, or some of the technical solutions may be implemented in a form of a software product. The software product is stored in a storage medium, and includes several instructions for instructing a computer device (which may be a personal computer, a server, or a network device) to perform all or some of the steps of the methods described in the embodiments of this application. The foregoing storage medium includes: any medium that can store program code, such as a USB flash drive, a removable hard disk, a read-only memory (Read-Only Memory, ROM), a random access memory (Random Access Memory, RAM), a magnetic disk, or a compact disc.

The foregoing descriptions are merely specific implementations of this application, but are not intended to limit the protection scope of this application. Therefore, the protection scope of this application shall be subject to the protection scope of the claims.

## Claims

1. An apnea monitoring method for simultaneous apnea monitoring of a plurality of users, comprising:
collecting (S210), by a multi-microphone array of a first terminal, a first sound of the plurality of users, wherein the plurality of users are located in coverage areas of different beams of the multi-microphone array;
obtaining (S220, S320, S430), based on the first sound, a power spectrum density corresponding to the first sound;
determining (S230, S330, S440) that at least one user encounters apnea when the power spectrum density is less than a first threshold;
wherein the collecting (S210) a first sound of the plurality of users comprises:
setting a sound pickup parameter of the multi-microphone array based on locations of the first terminal and the plurality of users, so that the different beams of the multi-microphone array cover the plurality of users; and
separately collecting, by the multi-microphone array, the first sound of the plurality of users in the coverage areas of the different beams;
wherein the determining that the at least one user encounters apnea when the power spectrum density is less than a first threshold comprises:
determining that the at least one user encounters apnea once when the duration during which the power spectrum density is less than the first threshold reaches a second threshold
and
sending (S510) a wake-up message to a second terminal by the first terminal, when determining that the duration during which the at least one user remains in apnea reaches a third threshold.

2. The apnea monitoring method according to claim 1, wherein the obtaining, based on the first sound, a power spectral density corresponding to the first sound comprises:
obtaining, based on the first sound, a time domain signal of the first sound; and
performing Fourier transformation on the time domain signal to obtain the power spectrum density of the first sound.

3. The apnea monitoring method according to any one of claims 1 to 2, wherein the method further comprises:
collecting, in advance, ambient noise of an environment in which the at least one user is located, and determining a frequency bandwidth of the ambient noise; and
filtering out the ambient noise based on the frequency bandwidth of the ambient noise.

4. The apnea monitoring method according to any one of claims 1 to 3, wherein the method further comprises:
determining, by using a voiceprint recognition algorithm, a first sound separately corresponding to each of at least one user.

5. The apnea monitoring method according to any one of claims 1 to 4, wherein the method further comprises:
determining sleep duration of the at least one user; and
determining an apnea-hypopnea index AHI of the at least one user based on the sleep duration and a quantity of times the at least one user encounters apnea in a sleep process.

6. An apnea monitoring terminal (600) for simultaneous apnea monitoring of a plurality of users, comprising:
a multi-microphone array;
at least one processor and one memory, wherein the memory is configured to store one or more computer programs; and when the one or more computer programs stored in the memory are executed by the at least one processor, the terminal is enabled to perform:
set a sound pickup parameter of the multi-microphone array based on locations of the first terminal and the plurality of users, so that the different beams of the multi-microphone array cover the plurality of users; and
separately collect (S220, S320, S430), by the multi-microphone array, a first sound of the plurality of users in the coverage areas of the different beams, wherein the plurality of users are located in coverage areas of different beams of the multi-microphone array;
obtain (S230, S330, S440), based on the first sound, a power spectrum density corresponding to the first sound; and
determine (S230, S330, S440) that at least one user encounters apnea when the power spectrum density is less than a first threshold; and
wherein the terminal is enabled to further perform:
determine that the at least one user encounters apnea once when the duration during which the power spectrum density is less than the first threshold reaches a second threshold;
send (S510) a wake-up message to a second terminal, when determining that the duration during which the at least one user remains in apnea reaches a third threshold.

7. The apnea monitoring terminal according to claim 6, the terminal is enabled to further perform:
obtain, based on the first sound, a time domain signal of the first sound; and
perform Fourier transformation on the time domain signal to obtain the power spectrum density of the first sound.

8. The apnea monitoring terminal according to any one of claims 6 to 7, the terminal is enabled to further perform:
collect, in advance, ambient noise of an environment in which the at least one user is located, and determine a frequency bandwidth of the ambient noise; and
filter out the ambient noise based on the frequency bandwidth of the ambient noise.

9. The apnea monitoring terminal according to any one of claims 6 to 8, the terminal is enabled to further perform:
determine, by using a voiceprint recognition algorithm, a first sound separately corresponding to each of at least one user.

10. The apnea monitoring terminal according to any one of claims 6 to 9, the terminal is enabled to further perform:
determine sleep duration of the at least one user; and
determine an apnea-hypopnea index AHI of the at least one user based on the sleep duration and a quantity of times the at least one user encounters apnea in a sleep process.

## Patentansprüche

1. Verfahren zum Überwachen von Apnoe für ein gleichzeitiges Überwachen von Apnoe einer Vielzahl von Benutzern, umfassend:
Sammeln (S210), durch ein Multimikrofon-Array eines ersten Endgeräts, eines ersten Tons der Vielzahl von Benutzern, wobei sich die Vielzahl von Benutzern in Abdeckungsbereichen von unterschiedlichen Strahlen des Multimikrofon-Arrays befinden;
Erhalten (S220, S320, S430), basierend auf dem ersten Ton, einer spektralen Leistungsdichte, die dem ersten Ton entspricht;
Ermitteln (S230, S330, S440), dass bei mindestens einem Benutzer eine Apnoe auftritt, wenn die spektrale Leistungsdichte kleiner als ein erster Schwellenwert ist;
wobei das Sammeln (S210) eines ersten Tons der Vielzahl von Benutzern umfasst:
Einstellen eines Tonaufnahmeparameters des Multimikrofon-Arrays basierend auf Orten des ersten Endgeräts und der Vielzahl von Benutzern, sodass die unterschiedlichen Strahlen des Multimikrofon-Arrays die Vielzahl von Benutzern abdecken; und
getrenntes Sammeln, durch das Multimikrofon-Array, des ersten Tons der Vielzahl von Benutzern in den Abdeckungsbereichen der unterschiedlichen Strahlen;
wobei das Ermitteln, dass bei mindestens einem Benutzer eine Apnoe auftritt, wenn die spektrale Leistungsdichte kleiner als ein erster Schwellenwert ist, umfasst:
Ermitteln, dass bei dem mindestens einen Benutzer einmal eine Apnoe auftritt, wenn die Dauer, während der die spektrale Leistungsdichte kleiner als der erste Schwellenwert ist, einen zweiten Schwellenwert erreicht, und
Senden (S510) einer Wecknachricht von dem ersten Endgerät an ein zweites Endgerät, wenn ermittelt wird, dass die Dauer, während der bei dem mindestens einen Benutzer die Apnoe bestehen bleibt, einen dritten Schwellenwert erreicht.

2. Verfahren zum Überwachen von Apnoe nach Anspruch 1, wobei das Erhalten, basierend auf dem ersten Ton, einer spektralen Leistungsdichte, die dem ersten Ton entspricht, umfasst:
Erhalten, basierend auf dem ersten Ton, eines Zeitbereichssignals des ersten Tons; und
Durchführen einer Fourier-Transformation an dem Zeitbereichssignal, um die spektrale Leistungsdichte des ersten Tons zu erhalten.

3. Verfahren zum Überwachen von Apnoe nach einem der Ansprüche 1 bis 2, wobei das Verfahren ferner umfasst:
vorheriges Sammeln eines Umgebungsrauschens in einer Umgebung, in der sich der mindestens eine Benutzer befindet, und Ermitteln einer Frequenzbandbreite des Umgebungsrauschens; und
Herausfiltern des Umgebungsrauschens basierend auf der Frequenzbandbreite des Umgebungsrauschens.

4. Verfahren zum Überwachen von Apnoe nach einem der Ansprüche 1 bis 3, wobei das Verfahren ferner umfasst:
Ermitteln, unter Verwendung eines Stimmabdruckerkennungsalgorithmus, eines ersten Tons, der jedem von mindestens einem Benutzer separat entspricht.

5. Verfahren zum Überwachen von Apnoe nach einem der Ansprüche 1 bis 4, wobei das Verfahren ferner umfasst:
Ermitteln einer Schlafdauer des mindestens einen Benutzers; und
Ermitteln eines Apnoe-Hypopnoe-Index AHI des mindestens einen Benutzers basierend auf der Schlafdauer und einer Häufigkeit, mit der bei dem mindestens einem Benutzer in einem Schlafprozess eine Apnoe auftritt.

6. Endgerät zum Überwachen von Apnoe (600) für ein gleichzeitiges Überwachen von Apnoe einer Vielzahl von Benutzern, umfassend:
ein Multimikrofon-Array;
mindestens einen Prozessor und einen Speicher, wobei der Speicher konfiguriert ist, um ein oder mehrere Computerprogramme zu speichern; und wobei, wenn das eine oder die mehreren Computerprogramme, die in dem Speicher gespeichert sind, von dem mindestens einen Prozessor ausgeführt werden, das Endgerät aktiviert wird zum Durchführen von:
Einstellen eines Tonaufnahmeparameters des Multimikrofon-Arrays basierend auf Orten des ersten Endgeräts und der Vielzahl von Benutzern, sodass unterschiedliche Strahlen des Multimikrofon-Arrays die Vielzahl von Benutzern abdecken; und
getrenntes Sammeln (S220, S320, S430), durch das Multimikrofonarray, eines ersten Tons der Vielzahl von Benutzern in den Abdeckungsbereichen der unterschiedlichen Strahlen, wobei sich die Vielzahl von Benutzern in Abdeckungsbereichen von unterschiedlichen Strahlen des Multimikrofon-Arrays befinden;
Erhalten (S230, S330, S440), basierend auf dem ersten Ton, einer spektralen Leistungsdichte, die dem ersten Ton entspricht; und
Ermitteln (S230, S330, S440), dass bei mindestens einem Benutzer eine Apnoe auftritt, wenn die spektrale Leistungsdichte kleiner als ein erster Schwellenwert ist; und
wobei das Endgerät aktiviert wird, um ferner durchzuführen:
Ermitteln, dass bei dem mindestens einen Benutzer einmal eine Apnoe auftritt, wenn die Dauer, während der die spektrale Leistungsdichte kleiner als der erste Schwellenwert ist, einen zweiten Schwellenwert erreicht;
Senden (S510) einer Wecknachricht an ein zweites Endgerät, wenn ermittelt wird, dass die Dauer, während der bei dem mindestens einen Benutzer die Apnoe bestehen bleibt, einen dritten Schwellenwert erreicht.

7. Endgerät zum Überwachen von Apnoe nach Anspruch 6, wobei das Endgerät aktiviert wird, um ferner durchzuführen:
Erhalten, basierend auf dem ersten Ton, eines Zeitbereichssignals des ersten Tons; und
Durchführen einer Fourier-Transformation an dem Zeitbereichssignal, um die spektrale Leistungsdichte des ersten Tons zu erhalten.

8. Endgerät zum Überwachen von Apnoe nach einem der Ansprüche 6 bis 7, wobei das Endgerät aktiviert wird, um ferner durchzuführen:
vorheriges Sammeln eines Umgebungsrauschens in einer Umgebung, in der sich der mindestens eine Benutzer befindet, und Ermitteln einer Frequenzbandbreite des Umgebungsrauschens; und
Herausfiltern des Umgebungsrauschens basierend auf der Frequenzbandbreite des Umgebungsrauschens.

9. Endgerät zum Überwachen von Apnoe nach einem der Ansprüche 6 bis 8, wobei das Endgerät aktiviert wird, um ferner durchzuführen:
Ermitteln, unter Verwendung eines Stimmabdruckerkennungsalgorithmus, eines ersten Tons, der jedem von mindestens einem Benutzer separat entspricht.

10. Endgerät zum Überwachen von Apnoe nach einem der Ansprüche 6 bis 9, wobei das Endgerät aktiviert wird, um ferner durchzuführen:
Ermitteln einer Schlafdauer des mindestens einen Benutzers; und
Ermitteln eines Apnoe-Hypopnoe-Index AHI des mindestens einen Benutzers basierend auf der Schlafdauer und einer Häufigkeit, mit der bei dem mindestens einem Benutzer in einem Schlafprozess eine Apnoe auftritt.

## Revendications

1. Procédé de surveillance d'apnée pour la surveillance simultanée d'apnées d'une pluralité d'utilisateurs, le procédé comprenant les étapes suivantes :
collecter (S210), par un réseau de plusieurs microphones d'un premier terminal, un premier son de la pluralité d'utilisateurs, où la pluralité d'utilisateurs se trouvent dans des zones de couverture de faisceaux différents du réseau de plusieurs microphones ;
obtenir (S220, S320, S430), sur la base du premier son, une densité spectrale de puissance correspondant au premier son ;
déterminer (S230, S330, S440) qu'au moins un utilisateur effectue une apnée lorsque la densité spectrale de puissance est inférieure à un premier seuil ;
où la collecte (S210) d'un premier son de la pluralité d'utilisateurs comprend les étapes suivantes :
régler un paramètre de prise de son du réseau de plusieurs microphones sur la base des emplacements du premier terminal et de la pluralité d'utilisateurs, de sorte que les différents faisceaux du réseau de plusieurs microphones couvrent la pluralité d'utilisateurs ; et
collecter séparément, par réseau de plusieurs microphones, le premier son de la pluralité d'utilisateurs dans les zones de couverture des différents faisceaux ;
où la détermination que l'au moins un utilisateur effectue une apnée lorsque la densité spectrale de puissance est inférieure à un premier seuil comprend les étapes suivantes :
déterminer que l'utilisateur effectue une fois une apnée lorsque la durée pendant laquelle la densité spectrale de puissance est inférieure au premier seuil atteint un deuxième seuil, et
envoyer (S510) un message de réveil à un deuxième terminal, par le premier terminal, lorsqu'il est déterminé que la durée pendant laquelle l'au moins un utilisateur reste en apnée atteint un troisième seuil.

2. Procédé de surveillance d'apnée selon la revendication 1, dans lequel l'obtention, sur la base du premier son, d'une densité spectrale de puissance correspondant au premier son comprend les étapes suivantes :
obtenir, sur la base du premier son, un signal dans le domaine temporel du premier son ; et
réaliser une transformation de Fourier sur le signal de domaine temporel afin d'obtenir la densité spectrale de puissance du premier son.

3. Procédé de surveillance d'apnée selon l'une quelconque des revendications 1 et 2, le procédé comprenant en outre les étapes suivantes :
collecter, à l'avance, un bruit ambiant d'un environnement dans lequel se trouve l'au moins un utilisateur, et déterminer une bande passante de fréquences du bruit ambiant ; et
filtrer le bruit ambiant sur la base de la largeur de bande de fréquences du bruit ambiant.

4. Procédé de surveillance d'apnée selon l'une quelconque des revendications 1 et 3, le procédé comprenant en outre l'étape suivante :
déterminer, au moyen d'un algorithme de reconnaissance d'empreinte vocale, un premier son correspondant séparément à chacun d'au moins un utilisateur.

5. Procédé de surveillance d'apnée selon l'une quelconque des revendications 1 et 4, le procédé comprenant en outre les étapes suivantes :
déterminer une durée de sommeil de l'au moins un utilisateur ; et
déterminer un indice d'apnée-hypopnée, AHI, de l'au moins un utilisateur sur la base de la durée de sommeil et d'une quantité de fois où l'au moins un utilisateur effectue une apnée au cours du sommeil.

6. Terminal de surveillance d'apnée (600) pour la surveillance simultanée d'apnées d'une pluralité d'utilisateurs, le terminal comprenant :
un réseau de plusieurs microphones ;
au moins un processeur et une mémoire, la mémoire étant configurée pour stocker un ou plusieurs programmes informatiques ; et, lorsque les un ou plusieurs programmes informatiques stockés dans la mémoire sont exécutés par l'au moins un processeur, le terminal peut exécuter les étapes suivantes :
régler un paramètre de prise de son du réseau de plusieurs microphones sur la base des emplacements du premier terminal et de la pluralité d'utilisateurs, de sorte que les différents faisceaux du réseau de plusieurs microphones couvrent la pluralité d'utilisateurs ; et
collecter séparément (S220, S320, S430), par le réseau de plusieurs microphones, un premier son de la pluralité d'utilisateurs dans les zones de couverture des différents faisceaux, où la pluralité d'utilisateurs sont situés dans des zones de couverture de différents faisceaux du réseau de plusieurs microphones ;
obtenir (S230, S330, S440), sur la base du premier son, une densité spectrale de puissance correspondant au premier son ; et
déterminer (S230, S330, S440) qu'au moins un utilisateur effectue une apnée lorsque la densité spectrale de puissance est inférieure à un premier seuil ; et
où le terminal peut en outre exécuter les étapes suivantes :
déterminer que l'utilisateur effectue une fois une apnée lorsque la durée pendant laquelle la densité spectrale de puissance est inférieure au premier seuil atteint un deuxième seuil ;
envoyer (S510) un message de réveil à un deuxième terminal lorsqu'il est déterminé que la durée pendant laquelle l'au moins un utilisateur reste en apnée atteint un troisième seuil.

7. Terminal de surveillance d'apnée selon la revendication 6, le terminal pouvant en outre exécuter les étapes suivantes :
obtenir, sur la base du premier son, un signal dans le domaine temporel du premier son ; et
réaliser une transformation de Fourier sur le signal de domaine temporel afin d'obtenir la densité spectrale de puissance du premier son.

8. Terminal de surveillance d'apnée selon l'une quelconque des revendications 6 à 7, le terminal étant autorisé à en outre exécuter les étapes suivantes :
collecter, à l'avance, un bruit ambiant d'un environnement dans lequel se trouve l'au moins un utilisateur, et déterminer une bande passante de fréquences du bruit ambiant ; et
filtrer le bruit ambiant sur la base de la largeur de bande de fréquences du bruit ambiant.

9. Terminal de surveillance d'apnée selon l'une quelconque des revendications 6 à 8, le terminal étant autorisé à en outre exécuter l'étape suivante :
déterminer, au moyen d'un algorithme de reconnaissance d'empreinte vocale, un premier son correspondant séparément à chacun d'au moins un utilisateur.

10. Terminal de surveillance d'apnée selon l'une quelconque des revendications 6 à 9, le terminal étant autorisé à en outre exécuter les étapes suivantes :
déterminer une durée de sommeil de l'au moins un utilisateur ; et
déterminer un indice d'apnée-hypopnée, AHI, de l'au moins un utilisateur sur la base de la durée de sommeil et d'une quantité de fois où l'au moins un utilisateur effectue une apnée au cours du sommeil.
